# EUROPÄISCHE PATENTANMELDUNG

(11) **EP 3 130 885 A1**
(43) Veröffentlichungstag der Anmeldung: **15.02.2017**
(21) Anmeldenummer: 15180555.3
(22) Anmeldetag: 11.08.2015
(51) Int. Cl.: G01B 11/00, A61B 5/107

(54) **SCANVORRICHTUNG, BALLON FÜR DEN BETRIEB MIT EINER SCANVORRICHTUNG, VERFAHREN ZUM BETRIEB EINER SCANVORRICHTUNG SOWIE STEUERPROGRAMM FÜR EINE SCANVORRICHTUNG**

(71) Anmelder: Ivoclar Vivadent AG, 9494 Schaan (LI)
(72) Erfinder: ROHNER, Gottfried, 9450 Altstätten (CH); WATZKE, Ronny, 6800 Feldkirch (DE); SENTI, Theresa, 9486 Schaanwald (LI)
(74) Vertreter: Baronetzky, Klaus

(57) **Zusammenfassung**

Die Erfindung betrifft eine Scanvorrichtung (10) mit einer Scankopfführung (18) für die Bewegung des Scankopfs (20) über einen Scanbereich (42) eines Hohlraums hinweg, welcher Scanbereich (42) sich um den Scankopf (20) erstreckt. Ein flächiges oder folienartiges, elastisch dehnbares Material erstreckt sich zwischen dem Scankopf (20) und dem Scanbereich (42), welches Material nach der Art eines Ballons (12) unter Anwendung von Überdruck an den Scanbereich (42) andrückbar ist. Die Scankopfführung (18) erstreckt sich durch den Ballonanschluss (16) des Ballons (12) zum Scankopf (20). Insbesondere eine Steuervorrichtung (32) ermittelt aus der Verformung des Materials beim Vorliegen des Überdrucks die Form des Hohlraums (40), an dem das Material anliegt.

## Beschreibung

Die Erfindung betrifft eine Scanvorrichtung gemäß dem Oberbegriff von Anspruch 1, einen Ballon gemäß dem Oberbegriff von Anspruch 15, ein Verfahren zum Betrieb einer Scanvorrichtung gemäß dem Oberbegriff von Anspruch 16 bzw. 19 sowie ein Steuerprogramm für eine Scanvorrichtung gemäß dem Oberbegriff von Anspruch 2229.

Es ist bekannt, um Aufnahmespulen für die Auswertung von Messsignalen Ballons anzuordnen, die sich aufblasen lassen. Das Aufblasen erfolgt über den Ballonanschluss, durch den hindurch auch die Anschlüsse für den Messaufnehmer geführt werden.

Eine derartige Lösung lässt sich für den medizinischen Bereich beispielsweise der DE 42 33 809 A1 entnehmen. Diese Lösung erlaubt den Schutz des empfindlichen Messaufnehmers, der beispielsweise eine NMR-Aufnahmevorrichtung sein kann.

In manchen Fällen ist es erwünscht, die innere Struktur eines teilweise auch zerklüfteten Hohlraums zu erfassen. Hierzu werden typischerweise stereometrische Verfahren verwendet, mit Scanköpfen, die voneinander um ein vorgegebenes Maß beabstandet sind. Über eine Beleuchtungsvorrichtung am Scankopf wird die zu erfassende Oberfläche des Hohlraums, der sogenannte Scanbereich, beleuchtet und der Scanbereich des Hohlraums soll so erfasst werden.

Häufig ist jedoch trotz aufwändiger Scanner das Scanergebnis unbefriedigend, so dass man versucht hat, entsprechend kostenaufwändig den Scankopf noch weiter zu verbessern. Dennoch lieferten die bislang insofern durchgeführten Versuche insbesondere bei dreidimensional kompliziert geformten Hohlräumen wenig befriedigende Ergebnisse.

Um dennoch eine einigermaßen präzise Erfassung der Oberfläche des Hohlraums, also des Scanbereichs zu ermöglichen, ist es bereits vorgeschlagen worden, mit unterschiedlichen Frequenzbereichen der elektromagnetischen Strahlung zu arbeiten. Bei einer feuchten Oberfläche kann es beispielsweise günstig sein, sichtbares Licht oder UV-Licht zu verwenden. Hingegen sind die Reflexionseigenschaften bestimmter Materialien, aus denen der Hohlraum bestehen kann, einerseits bei Ultraschall-, andererseits aber auch bei Röntgenstrahlung besser.

Nachteilig hierbei ist es, dass Scanner mit unterschiedlichen Frequenzbereichen bereitgestellt werden müssen, was die Lösung insgesamt verteuert und teilweise auch nicht mehr praktikabel macht.

Daher liegt der Erfindung die Aufgabe zugrunde, eine Scanvorrichtung gemäß dem Oberbegriff von Anspruch 1, einen Ballon für den Betrieb mit einer Scanvorrichtung gemäß dem Oberbegriff von Anspruch 15, ein Verfahren zum Betrieb einer Scanvorrichtung gemäß dem Oberbegriff von Anspruch 16 bzw. 19 sowie ein Steuerprogramm für eine Scanvorrichtung gemäß dem Oberbegriff von Anspruch 229 zu schaffen, die hinsichtlich des Scanergebnisses ohne nennenswerten Investitionsaufwand deutlich verbessert sind.

Diese Aufgabe wird erfindungsgemäß durch Anspruch 1, 15, 16, 19 bzw. 22 gelöst. Vorteilhafte Weiterbildungen ergeben sich aus den Unteransprüchen.

Erfindungsgemäß besonders günstig ist es, dass sich das folienartige oder flächige, elastisch dehnbare Material eng an den Scanbereich anlegt. Dies ist durch den Überdruck gewährleistet, der zudem bevorzugt steuerbar ist. Durch den Vergleich des Scanergebnisses mit einem geringen Überdruck und des Scanergebnisses mit einem größeren Überdruck ist es möglich, festzustellen, inwiefern der Scanbereich verformbar ist, also inwiefern er aus einer harten oder einer weichen Substanz besteht.

Wenn der Scanbereich bzw. der an den Scanbereich angrenzende Hohlraum gasförmige Einschlüsse aufweist, wird durch den Überdruck im Ballon die Substanz nach außen gedrückt. Wenn hingegen eine gallertartige, verformbare Masse vorliegt, wird die Oberfläche durch den Druck vergleichmäßigt, so dass sie also weniger zerklüftet als mit geringem Überdruck vorliegt.

Erfindungsgemäß ist es vorgesehen, dass der Scanbereich, der sich nahezu nach der Art eines zerklüfteten, aber im Wesentlichen kugelförmigen, Hohlraums um den Scankopf herum erstreckt, von dem dehnbaren Material, das Teil eines Ballons ist, vollständig abgedeckt wird.

Der Scankopf mit dem oder den Scannern wird durch den elastisch dehnbaren Ballonanschluss eingeführt, und anschließend hieran wird der Ballonanschluss gegenüber der Scankopfführung, die sich durch ihn hindurch erstreckt, abgedichtet.

Durch die Scankopfführung hindurch wird der Ballon nunmehr unter Druck gesetzt. Er legt sich hierdurch an den Scanbereich an.

Auf der Ballonfolie, die bevorzugt das elastisch dehnbare Material bildet, ist ein Referenzmuster aufgebracht. Dessen Ausgestaltung in entspanntem Zustand des Ballons ist bekannt. Durch die Ausdehnung des Ballons verformt sich das Referenzmuster, so dass sich die einzelnen Referenzlinien und Referenzpunkte voneinander weg bewegen. Aus dieser Dehnung, die je punktuell erfolgt, kann nun mittelbar auf die Form des Hohlraums en detail geschlossen werden, ohne dass es einer fehlerbehafteten stereoskopischen Untersuchung bedarf.

Das Referenzmuster kann in beliebiger geeigneter Weise auf der Ballonfolie aufgebracht sein. Bei der Aufbringung von innen ist es günstig, wenn die Ballonfolie dünn ist, beispielsweise 80 µm, um die Genauigkeit der Messung aufrechtzuerhalten. Bei Aufbringung außen ist eine volle Transparenz der Ballonfolie günstig, beispielsweise mit einem Transmissionsgrad von 0,9 oder sogar einem größeren Transmissionsgrad. Auch hier ist insofern eine dünne Ballonfolie günstig.

Die Steuervorrichtung der erfindungsgemäßen Scanvorrichtung erfasst die von dem oder den Scannern aufgenommene Dehnung des Referenzmusters an jeder Stelle des Scanbereichs. Aus dieser Dehnung wird nun die dreidimensionale Form des Scanbereichs an der betrachteten Stelle berechnet, wobei der Erfassung zugute kommt, dass die Dehnung zweidimensional erfolgt und auch zweidimensional erfasst werden kann. Insofern ist es für das Referenzmuster wichtig, dass ein echtes Muster vorliegt, also nicht nur sich nebeneinander erstreckende Linien ohne Kreuzung.

Bei der Auswahl des Musters ist es günstig, die software-mäßige Erkennbarkeit zu optimieren. Beispielsweise kann ein geometrisch gleichmäßiges Muster mit strichpunktierten Linien in eine Richtung und gestrichelten Linien in der Querrichtung hierzu günstig sein. Es sind aber auch beliebige andere regelmäßige aber auch unregelmäßige Referenzmuster realisierbar. Ein Beispiel ist ein Hexagonalmuster mit einer Schenkellänge von 200µm.

Die Strichstärke des Referenzmusters lässt sich in weiten Bereichen an die Auflösung der Scanner des Scankopfes anpassen. So kann beispielsweise eine sehr feine Strichstärke von 15µm eine sehr genauer Erfassung ermöglichen.

In vorteilhafter Ausgestaltung ist es vorgesehen, dass das Material als elastische Kunststofffolie ausgebildet ist, die insbesondere wasser- und/oder fettbeständig ist und bevorzugt aus Elastomer, Silikon oder Latex besteht. Das elastisch dehnbare Material kann als Kunststofffolie und als Wegwerfteil ausgebildet sein.

Der Scanbereich kann auch unmittelbar mit einem Referenzmuster versehen sein, z.B. indem ein deartiges Muster optisch auf den Scanbereich geworfen wird.

Bevorzugt ist die Ballonfolie transparent ausgebildet und weist insbesondere eine Stärke von weniger als 300µm, bevorzugt etwa 50µm, auf.

In weiterer vorteilhafter Ausgestaltung ist es vorgesehen, dass eine Überdruckquelle vorgesehen ist, die einen im Wesentlichen konstanten Druck abgibt und an den Ballonanschluss angeschlossen ist, wobei mit dem Ballonanschluss oder der Überdruckquelle insbesondere ein Überdruckventil in Strömungsverbindung steht.

In weiterer vorteilhafter Ausgestaltung ist es vorgesehen, dass die Ballonfolie, beispielsweise durch Anlegen von Unterdruck kollabierbar und durch den Ballonanschluss aus dem Hohlraum entfernbar ist.

In weiterer vorteilhafter Ausgestaltung ist es vorgesehen, dass die Ballonfolie beim Anlegen von Überdruck außerhalb des Hohlraums eine Kugelform einnimmt, mit einer gegenüber der Kugelform vorspringenden kalottenförmigen Außenform in Richtung des Ballonanschlusses.

In weiterer Ausgestaltung der Erfindung ist ein Verfahren zum Betrieb einer Scanvorrichtung vorgesehen, die einen Scankopf und eine Scankopfführung aufweist, mit einem für die Scanstrahlung transparenten Scanhilfsmittel, wobei das Verfahren dadurch gekennzeichnet ist, dass das Scanhilfsmittel in weichem oder flüssigem Zustand in einen zu scannenden Hohlraum eingeführt wird, dass unter Aushärten des Scanhilfsmittels oder während des Aushärtens des Scanhilfsmittels der Hohlraum verkleinert wird, dass nach dem Aushärten das Scanhilfsmittel entnommen wird, dass dann der Scankopf in das Scanhilfsmittel eingeführt wird und die Oberfläche des Scanhilfsmittels gescannt wird.

Erfindungsgemäß wesentlich ist es, dass während des Scanvorgangs die Position des Scankopfes relativ zu dem Hohlraum nicht verändert wird. Dies lässt sich beispielsweise durch ein Referenzobjekt realisieren, das in dem Ballon und einem unwichtigen Bereich des Hohlraums benachbart angeordnet ist. Bei Bewegung des Scankopfes erfolgt dann auch eine Bewegung relativ zum Referenzobjekt, die dann wiederum herausgerechnet werden kann.

Ein derartiges Referenzobjekt, von dem auch mehrere über den Hohlraum verteilt angeordnet sein können, kann auch zur verbesserten Aneinanderreihung der erfassten Scanbilder dienen, dem sogenannten Stitching. Das Referenzobjekt kann auch gegebenenfalls lösbar innen an der Folie angebracht sein.

Erfindungsgemäß ist eine stereometrische Erfassung des Scanbereichs bzw. indirekt des dort anliegenden Referenzmusters nicht erforderlich. Es kann jedoch zur Verbesserung der Genauigkeit günstig sein, mindestens einen Punkt des Referenzmusters trigonometrisch von zwei voneinander beabstandeten Scannern zu erfassen.

Das elastisch dehnbare Material kann Teil eines Ballons sein, der mit dem Referenzmuster bedruckt ist. Der Druck ist preisgünstig zu realisieren, gerade wenn eine Anbringung auf der Außenseite der Ballonfolie realisiert wird. In diesem Fall kann der Ballon auch als Wegwerfteil ausgebildet sein, der kurzerhand über den Scankopf gestülpt wird.

In weiterer vorteilhafter Ausgestaltung ist es vorgesehen, den Ballon zunächst unter einen begrenzten Überdruck zu setzen, der auch steuerbar sein kann, beispielsweise über ein Überdruckventil. Die eigentliche Dehnung des Referenzmusters, das auf der Ballonfolie aufgebracht ist, erfolgt dann durch eine gezielte Volumenverkleinerung des Hohlraums, beispielsweise dann, wenn dieser aus zwei einander gegenüberliegenden Halbschalen besteht.

In weiterer vorteilhafter Ausgestaltung ist es vorgesehen, dass die Scanvorrichtung die Volumenvergrößerung des Ballons und/oder die Dehnung der Ballonfolie beim Aufblasen erfasst. Aus dieser Dehnung läßt sich auf die Stärke der Ballonfolie nach dem Aufblasen an der je betrachteten Stelle schließen, da die Dehnung in ersten Näherung umgekehrt proportional zur Wandstärke ist.

Hierzu ermittelt die Steuervorrichtung die Vergrößerung des Referenzmuisters beim Aufblasen. Die Ballonfoliewird gegenüber einer angenäherten Kugelform verformt, sobald sie an dem Scanbereich in Anlage gerät. Bis zu diesem Punkt erfolgt eine im wesentlichen gleichmäßige Dehnung der Ballonfolie, und eine entsprechende 2-dimensionale Vergrößerung des Referenzmusters, .die von der Scanvorrichtung erfaßt wird.

In gleichem Maße wird die Ballonfolie dünner, so dass bei einer bekannten Ausgangs-Wandstärke von z.B. 150 µm und einer - linearen - Referenzmuster-Vergrößerung um den Faktor 3 die verbleibende Ballonfolien-Wandstärke auf 50 µm berechnet werden kann.

Sobald die Ballonfolie in einer Stelle in Anlage an den Scanbereich gerät, wird das Referenzmuster an dieser Stelle nicht wesentlich weiter vergrößert. Dies wird erfindungsgemäß erfasst.

Für die Erfassung der Dehnung im Raum wird in der Steuervorrichtung zweckmäßig ein Polarkordinatensystem zugrunde gelegt.

Wenn der Druck im Ballon weiter erhöht wird, und z.B. wenn der Ballon allseitig am Scanbereich anliegt, erfolgt jedoch eine geringe Weitervergrößerung des Referenzmusters, die der Nachgiebigkeit des Scanbereichs an dieser Stelle entspricht.

Aus dem Quotienten von Druckzunahme - entsprechend der aufgebrachten Kraft auf die betrachtete Stelle des Scanbereichs - und Dehnung in tangentialer Richtung - entsprechend dem Weg in radialer Richtung - läßt sich die Verformung des Scanbereichs an jeder Stelle durch die aufgebrachte Kraft - und damit, soweit die Geltung des Hookeschen Gesetzes angenommen werden kann, dessen dortiger E-Modul, ermitteln.

Zudem läßt sich die Reduktion der Wandstärke des Ballonfolie - soweit erforderlich - in die Berechnung einbeziehen. Die Wandstäke ist insbesondere dann von Bedeutung, wenn das Referenzmuster auf der Innenseite des Ballons aufgebracht ist, da der Scanbereich dann in radialer Richtung um die Wandstärke von dem Referenzmuster entfernt ist.

Beim Aufdruck auf die Außenseite des Ballons wird dieses aber ebenfalls tangential gedehnt und dadurch dünner; auch diese Dickenreduktion läßt sich bei Bedarf in die Berechnung einbeziehen.

Erfindungsgemäß ist eine Scannvorrichtung mit einer Auflösung und Genauigkeit zwischen 10 und 50 µm bevorzugt. Das aufgedruckte Referenzmuster kann eine Stärke von 30 µm haben und erfährt eine Dickenreduktion auf 10 µm bei einer tangential-linearen Ballondehnung um den Faktor 3. Die Scanvorrichtung erfasst die radiale Innenseite des Aufdrucks, so dass der Scanbereich um 10µm radial weiter außen ist, als die Erfassung durch die Scanvorrichtung ergibt. Insofern fällt diese Differenz nur bei Scanvorrichtungen mit sehr hoher Genauigkeit ins Gewicht, und kann typischerweise vernachlässigt werden.

Weitere Vorteile, Einzelheiten und Merkmale ergeben sich aus der nachfolgenden Beschreibung mehrerer Ausführungsbeispiele der Erfindung anhand der Zeichnung.

Es zeigen:
- Fig. 1: eine schematische Ansicht einer erfindungsgemäßen Ausführungsform einer Scanvorrichtung;
- Fig. 2: ein Detail aus Fig. 1 in zwei Zuständen; und
- Fig. 3: eine weitere Ausführungsform eines erfindungsgemäßen Details der Scanvorrichtung, nämlich des Ballons mit dem Referenzmuster und einer Zugvorrichtung.

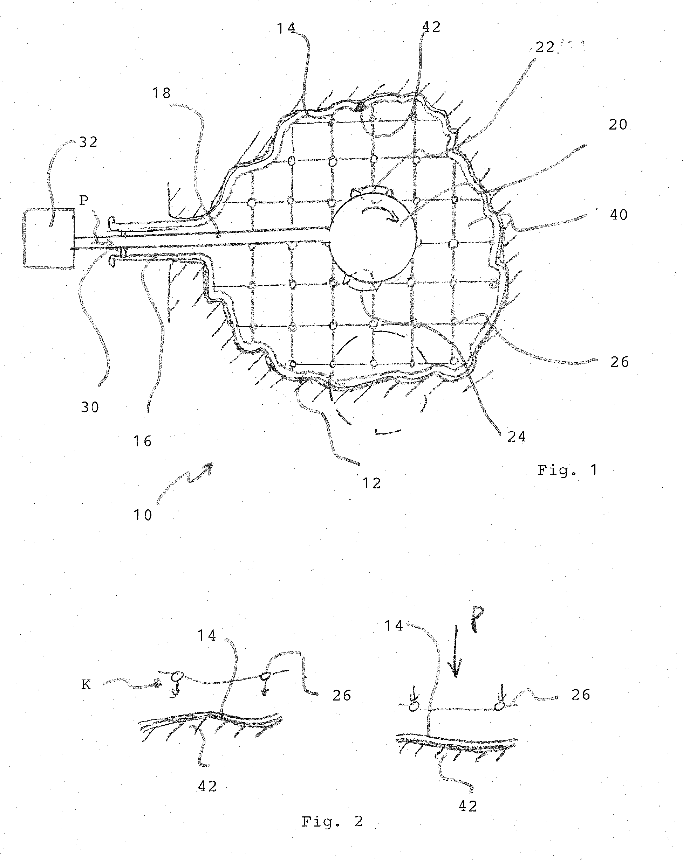

Aus Fig. 1 ist eine erfindungsgemäße Scanvorrichtung 10 in schematischer Darstellung ersichtlich. Sie weist einen Ballon 12 auf, der aus einer Ballonfolie 14 besteht, die das elastisch dehnbare Material bildet. Der Ballon 12 hat in an sich bekannter Weise einen Ballonanschluss 16. Durch diesen hindurch erstreckt sich eine Scankopfführung 18, die einen Scankopf 20 im Inneren des Ballons hält und sicher führt.

An dem Scankopf 20 sind mehrere Scanner angebracht, von denen hier zwei Scanner 22 und 24 dargestellt sind. Tatsächlich kann auch eine Vielzahl von Scannern vorgesehen sein, beispielsweise 100, während bei einer geringen Anzahl von Scannern diese bevorzugt an dem Scankopf 20 beweglich gelagert sind.

Die Scanner haben die Aufgabe, den gesamten Innenraum der Ballonfolie 14 zu erfassen. Die Ballonfolie 14 weist ein Referenzmuster 26 auf, das im dargestellten Ausführungsbeispiel nach der Art eines Netzes ausgebildet ist, das sich im gleichmäßigen Linienabstand über die Ballonfolie erstreckt.

Die Scankopfführung 18 umgebend ist eine Dichtung 30 vorgesehen, die den Ballonanschluss 16 gegenüber der Umgebungsluft abdichtet. Eine Steuervorrichtung 32 ist außerhalb des Ballons vorgesehen. Diese wertet jedenfalls die von den Scannern erfassten Bilder aus und steuert in dem dargestellten Ausführungsbeispiel auch einen Überdruck P, mit dem der Ballon 12 aufgeblasen wird.

Durch das Aufblasen legt sich die Ballonfolie 14 eng und genau der Kontur folgend an einen Hohlraum 40 an. Das Referenzmuster 26 erstreckt sich so entlang eines Scanbereichs 42 in verformtem Zustand. Aus der Verformung des Referenzmusters 26 lässt sich erfindungsgemäß die Form des Scanbereichs im Einzelnen berechnen.

Die erfindungsgemäße Lösung ist auch besonders für die Erfassung der Verformbarkeit des Hohlraums geeignet. Hierzu sei auf Fig. 2 verwiesen. Der dort dargestellte Ausschnitt der Ballonfolie 14 wird mit der Kraft K, die von der Größe des Überdrucks P abhängt, an den Scanbereich 42 angedrückt. In dem in Fig. 42 links dargestellten Zustand legt sich die Ballonfolie 14 der Kontur des Scanbereichs 42 folgend an diese an, und es erfolgt eine entsprechende Verformung des Referenzmusters 26.

Wenn ein höherer Druck P ausgeübt wird, wird der elastische Scanbereich 42 weiter nach unten verdrängt, und das Referenzmuster 26 erstreckt sich weniger stark strukturiert, woraus sich die elastische Verformbarkeit entnehmen lässt.

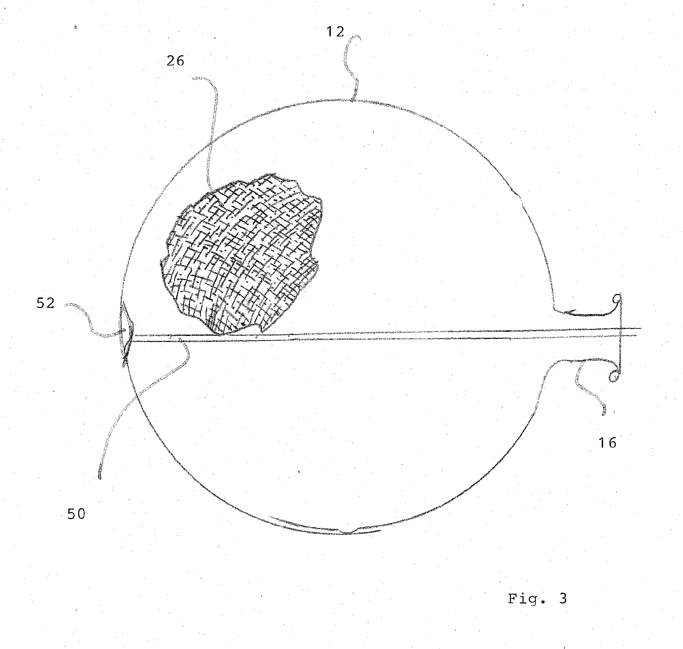

Aus Fig. 3 ist ersichtlich, in welcher Weise in einer modifizierten Ausgestaltung das Referenzmuster 26 an dem Ballon 12 angebracht sein kann. Auch wenn hier der Einfachheit der Darstellung halber das Referenzmuster mit einem Linienabstand von beispielsweise 500µm realisiert dargestellt ist, versteht es sich, dass in der Praxis wesentlich feinere Muster möglich sind, beispielsweise mit einem Strukturabstand von 50µm und einer Strichstärke von 15µm.

Wie aus Fig. 3 ersichtlich ist, ist in einer Dimension eine strichpunktierte Linienausgestaltung gewählt, und in der anderen Dimension eine gestrichelte. Hieraus lässt sich über den Scankopf 20 die relative Ausrichtung im Raum erfassen.

In dem dargestellten Ausführungsbeispiel gemäß Fig. 3 erstreckt sich eine Zugvorrichtung 50 von dem dem Ballonanschluss 12 gegenüberliegenden entfernten Bereich 52 des Ballons durch den Ballonanschluss 16 hindurch, um diesen Bereich 52 von dem Hohlraum 40 gezielt zu trennen.

Diese Lösung ist beispielsweise dann günstig, wenn der Hohlraum der Mund eines Patienten ist und die Abtastung des Mundinnenraums erfolgen soll, ohne dass ein Würgereiz durch den Überdruck des Ballons entsteht.

In vorteilhafter Ausgestaltung wird der Ballon 12 so aufgeblasen, dass er an dem Hohlraum innen vollständig anliegt. Durch Volumenverkleinerung des Hohlraums erfolgt dann eine weitere Druckzunahme, die über den Scankopf 20 erfassbar ist.

Der Scankopf 20 kann in einem beliebigen geeigneten Wellenlängenbereich betrieben werden. Zu denken ist hier an elektromagnetische Strahlung wie Lichtstrahlung, wozu eine zusätzliche Lichtquelle bevorzugt ist, die den Balloninnenraum ausleuchtet und an dem Scankopf 20 angebracht ist. Auch die Verwendung von Röntgenstrahlung oder von Ultraschall ist anstelle dessen oder zusätzlich möglich.

In einer weiteren Ausführungsform ist es vorgesehen, zunächst ein für die Scanstrahlung transparentes Scanhilfsmittel in den Hohlraum einzuführen und dann aushärten zu lassen.

Das Scanhilfsmittel weist eine Einführöffnung für einen Scankopf auf. Im Hohlraum 40 wird es ausgehärtet, wobei sich das auf der Oberfläche des Scanhilfsmittels aufgebrachte Referenzmuster verformt. Nach einem mindestens teilweisen Aushärten wird es - gegebenenfalls unter elastischem Zusammendrücken oder unter Öffnen des Hohlraums - entnommen, und hieran anschließend wird der Scankopf in das Scanhilfsmittel eingeführt und die Oberfläche des Scanhilfsmittels von innen gescannt.

## Patentansprüche

1. Scanvorrichtung mit einer Scankopfführung für die Bewegung des Scankopfs über einen Scanbereich eines Hohlraums hinweg, welcher Scanbereich sich um den Scankopf erstreckt, **dadurch gekennzeichnet, dass** ein flächiges oder folienartiges, elastisch dehnbares Material sich zwischen dem Scankopf (20) und dem Scanbereich (42) erstreckt, welches Material nach der Art eines Ballons (12) unter Anwendung von Überdruck an den Scanbereich (42) andrückbar ist, und dass die Scankopfführung (18) sich durch den Ballonanschluss (16) des Ballons (12) zum Scankopf (20) erstreckt, und dass insbesondere eine Steuervorrichtung (32) aus der Verformung des Materials beim Vorliegen des Überdrucks die Form des Hohlraums (40), an dem das Material anliegt, ermittelt.

2. Scanvorrichtung nach einem der vorhergehenden Ansprüche, **dadurch gekennzeichnet, dass** das elastisch dehnbare Material mit einem Referenzmuster (26) versehen ist, das insbesondere auf dem Material innen oder außen - bezogen auf den Ballon (12) - aufgebracht ist.

3. Scanvorrichtung nach einem der vorhergehenden Ansprüche, **dadurch gekennzeichnet, dass** ein flexibles Netz oder ein flexibler Gitterrahmen vorgesehen ist, der an den Scanbereich (42) angelegt ist und von dem elastisch dehnbaren Material an den Scanbereich (42) angedrückt wird, welches oder welcher als Referenzmuster (26) dient.

4. Scanvorrichtung nach einem der vorhergehenden Ansprüche, **dadurch gekennzeichnet, dass** der Scanbereich (42) mit einem Referenzmuster (26) versehen ist.

5. Scanvorrichtung nach einem der vorhergehenden Ansprüche, **dadurch gekennzeichnet, dass** eine Steuervorrichtung (32) für den Überdruck vorgesehen ist, die den Überdruck erhöht, bis sich das Referenzmuster (26) beim Scannen um nicht mehr als ein vorgegebenes Maß ändert.

6. Scanvorrichtung nach einem der vorhergehenden Ansprüche, **dadurch gekennzeichnet, dass** die Ballonfolie (14) an der dem Ballonanschluss (16) gegenüberliegenden Seite eine Zugvorrichtung (50) aufweist, durch welche die Folie bei Überdruck in Richtung Ballonanschluss (16) ziehbar und/oder an dem Ballonanschluss (16) fixierbar ist.

7. Scanvorrichtung nach einem der vorhergehenden Ansprüche , **dadurch gekennzeichnet, dass** eine Ballonfolie (14) an einem dem Ballonanschluss (16) gegenüberliegenden Bereich ausgesteift und in Richtung des Ballonanschlusses (16) vorgespannt ist, so dass ohne Überdruck dort eine Kalotte entsteht, die auch beim Überdruck bestehen bleibt.

8. Scanvorrichtung nach einem der vorhergehenden Ansprüche, **dadurch gekennzeichnet, dass** die Ballonfolie (14) eine vom Ballonanschluss (16) ausgehend abnehmende Wandstärke aufweist, insbesondere von 200µm auf weniger als 100µm, bevorzugt auf 50µm, und eine hieran anschließend zunehmende Wandstärke bis zu der dem Ballonanschluss (16) gegenüberliegenden Stelle, wobei die maximale Wandstärke weniger als 500µm beträgt.

9. Scanvorrichtung nach einem der vorhergehenden Ansprüche, **dadurch gekennzeichnet, dass** der Scankopf (20) mindestens zwei voneinander beabstandete Scanner (22, 24) aufweist, die den Abstand zu mindestens einem Punkt des Referenzmusters (26) trigonometrisch erfassen.

10. Scanvorrichtung nach einem der vorhergehenden Ansprüche, **dadurch gekennzeichnet, dass** die Ballonfolie (14) für eine von dem Scankopf (20) abgegebene Strahlung und/oder für eine von dem Scanbereich (42) reflektierte Strahlung transparent ist, insbesondere mit einem Transmissionsgrad von mehr als 0,8.

11. Scanvorrichtung nach einem der vorhergehenden Ansprüche, **dadurch gekennzeichnet, dass** das Referenzmuster (26) eine von dem Scankopf (20) abgegebene Strahlung absorbiert, mit einem Absorptionskoeffizienten von 100/mm und einem Reflexionsgrad von weniger als 5%.

12. Scanvorrichtung nach einem der vorhergehenden Ansprüche, **dadurch gekennzeichnet, dass** das Referenzmuster (26) eine Stärke von weniger als 50% der Stärke der Folie aufweist, insbesondere weniger als 10% dieser.

13. Scanvorrichtung nach einem der vorhergehenden Ansprüche, **dadurch gekennzeichnet, dass** das Referenzmuster (26) eine unregelmäßige geometrische Struktur aufweist, insbesondere mit mehreren voneinander unterscheidbaren Aufdrucken, welche sich insbesondere über einen überwiegenden Teil des elastisch dehnbaren Materials hinweg im Raum erstrecken.

14. Scanvorrichtung nach einem der vorhergehenden Ansprüche, **dadurch gekennzeichnet, dass** das Referenzmuster (26) eine Mehrzahl von Spezialreferenzpunkten aufweist, die vorab zu markanten Punkten des Hohlraums (40) ausgerichtet werden, insbesondere durch manuelles Verschieben des elastisch dehnbaren Materials zu den Punkten hin.

15. Ballon für den Betrieb mit einer Scanvorrichtung, mit einem Ballonanschluss, über welchen der Ballon unter Überdruck setzbar ist, **gekennzeichnet durch** ein Referenzmuster (26), das auf mindestens einem Teil des Ballons (12), insbesondere auf der inneren Oberfläche des Ballons (12), angebracht ist.

16. Verfahren zum Betrieb einer Scanvorrichtung, , **dadurch gekennzeichnet, dass** ein Ballon (12) aus einer dehnbaren Folie, auf der ein Referenzmuster (26) angebracht ist, über einen insbesondere mindestens 3-achsig beweglich gelagerter Scankopf (20) der Scanvorrichtung (10) gestülpt wird und diese beiden in einen insbesondere verformbaren
Hohlraum (40) eingeführt werden, dass dann der Ballon (12) über seinen Ballonanschluss (16) unter Überdruck gesetzt wird, so dass er sich an den Hohlraum (40) anlegt, und dass dann ein Scan über einen vorgegebenen Scanbereich (42) hinweg gestartet und durchgeführt wird.

17. Verfahren nach Anspruch 16, **dadurch gekennzeichnet, dass** ein erster Scan bei einem ersten Überdruck, bei welchem die Ballonfolie (14) an dem Hohlraum (40) anliegt, vorgenommen wird und eine zweiter Scan bei einem zweiten Überdruck, bei welchem die Ballonfolie (14) den Hohlraum (40) verformt, vorgenommen wird.

18. Verfahren nach Anspruch 16 oder 17, **dadurch gekennzeichnet, dass** eine Steuervorrichtung (32) der Scanvorrichtung (10) aus der Verformung des Referenzmusters (26) entsprechend der Verformung der Oberfläche des Hohlraums (40) die Nachgiebigkeit der Oberfläche und/oder die Veränderung der Oberfläche ermittelt.

19. Verfahren zum Betrieb einer Scanvorrichtung, mit einem Scankopf und einer Scankopfführung, die durch eine Öffnung in einem Hohlraum geführt wird, **dadurch gekennzeichnet, dass** der Hohlraum (40) mit einem elastisch dehnbaren, sich über die Fläche betrachtet zwei-dimensional erstreckenden oder folienartigen Material abgedeckt ist, das an den Hohlraum (40) angedrückt wird, und **dadurch gekennzeichnet, dass** der Hohlraum (40) vor dem Scannen in seinem Volumen verkleinert wird.

20. Verfahren nach Anspruch 19, **dadurch gekennzeichnet, dass** der Hohlraum (40) kontrolliert hinsichtlich seines Volumens verkleinert wird und insbesondere die Form des Referenzmusters (26) des folienartigen Materials erfasst und/oder gemessen wird.

21. Verfahren nach einem der Ansprüche 16 bis 20, **dadurch gekennzeichnet, dass** die Scanvorrichtung die Volumenvergrößerung des Ballons und/oder die Dehnung der Ballonfolie (14) beim Aufblasen erfasst und hierzu die Steuervorrichtung (32) die Vergrößerung des Referenzmusters (26) beim Aufblasen, ermittelt, insbesondere bevor die Ballonfolie (14) duch Anlage an den Scanbereich (42) verformt wird und, nachdem die Ballonfolie durch Anlage an den Scanbereich verformt wird, und insbesondere hieraus die Reduktion der Stärke der Ballonfolie und/oder des Referenzmusters (26) errechnet.

22. Steuerprogramm für eine Scanvorrichtung, **dadurch gekennzeichnet, dass** es das Verfahren gemäß einem der Ansprüche 16 bis 21 ausführt.

## Geänderte Patentansprüche

### Geänderte Patentansprüche gemäss Regel 137(2) EPÜ.

1. Scanvorrichtung (18) mit einer Scankopfführung für die Bewegung des Scankopfs über einen Scanbereich eines Hohlraums (40) hinweg, welcher Scanbereich (42) sich um den Scankopf (20) erstreckt, wobei ein flächiges oder folienartiges, elastisch dehnbares Material vorgesehen ist, welches sich zwischen dem Scankopf (20) und dem Scanbereich (42) erstreckt, und nach der Art eines Ballons (12) unter Anwendung von Überdruck an den Scanbereich (42) andrückbar ist, wobei der Ballon (12) einen Ballonanschluss (16) aufweist, durch welchen sich eine Scankopfführung (18) zum Scankopf (20) hin erstreckt, **dadurch gekennzeichnet, dass** der Ballon (12), welcher aus einer Ballonfolie (14) besteht, ein nach der Art eines Netzes ausgebildetes und sich über die Ballonfolie (14) erstreckendes Referenzmuster (26), das sich im gleichmäßigen Linienabstand über die Ballonfolie erstreckt, aufweist, so dass sich das Referenzmuster (26) durch das Aufblasen der Ballonfolie (14) entlang des Scanbereichs (42) im verformten Zustand erstreckt, wobei aus der Verformung des Referenzmusters (26) die Form des Scanbereichs (42) des Hohlraums (40) ermittelbar ist.

2. Scanvorrichtung nach einem der vorhergehenden Ansprüche, **dadurch gekennzeichnet, dass** das Referenzmuster (26) auf dem Material innen oder außenbezogen auf den Ballon (12) - aufgebracht ist.

3. Scanvorrichtung nach einem der vorhergehenden Ansprüche, **dadurch gekennzeichnet, dass** eine Steuervorrichtung (32) außerhalb des Ballons (12) vorgesehen ist, zum Steuern des Überdrucks mit dem der Ballon (12) aufgeblasen wird, zum Auswerten der gescanten Bilder, wobei aus der Verformung des Referenzmusters (26) die Form des Scanbereichs (42) berechenbar ist.

4. Scanvorrichtung nach einem der vorhergehenden Ansprüche, **dadurch gekennzeichnet, dass** die Steuervorrichtung (32) den Überdruck erhöht, bis sich das Referenzmuster (26) beim Scannen um nicht mehr als ein vorgegebenes Maß ändert.

5. Scanvorrichtung nach einem der vorhergehenden Ansprüche, **dadurch gekennzeichnet, dass** die Ballonfolie (14) an der dem Ballonanschluss (16) gegenüberliegenden Seite eine Zugvorrichtung (50) aufweist, durch welche die Folie bei Überdruck in Richtung Ballonanschluss (16) ziehbar und/oder an dem Ballonanschluss (16) fixierbar ist.

6. Scanvorrichtung nach einem der vorhergehenden Ansprüche, **dadurch gekennzeichnet, dass** die Ballonfolie (14) beim Anlegen von Überdruck außerhalb des Hohlraums (40) eine Kugelform einnimmt, mit einer gegenüber der Kugelform vorspringenden kalottenförmigen Außenform in Richtung des Ballonanschlusses (16), die auch beim Überdruck bestehen bleibt.

7. Scanvorrichtung nach einem der vorhergehenden Ansprüche, **dadurch gekennzeichnet, dass** aus der Dehnung der Ballonfolie (14) eine vom Ballonanschluss (16) ausgehend abnehmende Wandstärke aufweist, insbesondere von weniger als 300 µm, bevorzugt etwa 50µm, wobei die maximale Wandstärke weniger als 500µm beträgt.

8. Scanvorrichtung nach einem der vorhergehenden Ansprüche, **dadurch gekennzeichnet, dass** der Scankopf (20) mindestens zwei voneinander beabstandete Scanner (22, 24) aufweist, die den Abstand zu mindestens einem Punkt des Referenzmusters (26) trigonometrisch erfassen.

9. Scanvorrichtung nach einem der vorhergehenden Ansprüche, **dadurch gekennzeichnet, dass** die Ballonfolie (14) für eine von dem Scankopf (20) abgegebene Strahlung und/oder für eine von dem Scanbereich (42) reflektierte Strahlung transparent ist, mit einem Transmissionsgrad von mehr als 0,8.

10. Scanvorrichtung nach einem der vorhergehenden Ansprüche, **dadurch gekennzeichnet, dass** das Referenzmuster (26) eine von dem Scankopf (20) abgegebene Strahlung absorbiert, mit einem Absorptionskoeffizienten von 100/mm und einem Reflexionsgrad von weniger als 5%.

11. Scanvorrichtung nach einem der vorhergehenden Ansprüche, **dadurch gekennzeichnet, dass** das Referenzmuster (26) eine Stärke von weniger als 50% der Stärke der Folie aufweist, vorzugsweise weniger als 10% dieser.

12. Scanvorrichtung nach einem der vorhergehenden Ansprüche, **dadurch gekennzeichnet, dass** das Referenzmuster (26) eine unregelmäßige geometrische Struktur aufweist, insbesondere mit mehreren voneinander unterscheidbaren Aufdrucken, welche sich über einen überwiegenden Teil des elastisch dehnbaren Materials hinweg im Raum erstrecken.

13. Scanvorrichtung nach einem der vorhergehenden Ansprüche, **dadurch gekennzeichnet, dass** das Referenzmuster (26) eine Mehrzahl von Spezialreferenzpunkten aufweist, die vorab zu markanten Punkten des Hohlraums (40) durch manuelles Verschieben des elastisch dehnbaren Materials zu den Punkten hin ausgerichtet werden können.

14. Ballon für den Betrieb mit einer Scanvorrichtung nach den Ansprüchen 1 bis 13, mit einem Ballonanschluss, über welchen der Ballon unter Überdruck setzbar ist, **gekennzeichnet durch** ein Referenzmuster (26), das auf mindestens einem Teil des Ballons (12), auf der inneren Oberfläche des Ballons (12), angebracht ist.

15. Verfahren zum Betrieb einer Scanvorrichtung, **dadurch gekennzeichnet, dass** ein Ballon (12) aus einer dehnbaren Folie (14), auf der ein Referenzmuster (26), welches nach der Art eines Netzes ausgebildet ist und sich im gleichmäßigen Linienabstand über die Ballonfolie (14) erstreckt, angebracht ist, über einen insbesondere mindestens 3-achsig beweglich gelagerter Scankopf (20) der Scanvorrichtung (10) gestülpt wird und diese beiden in einen verformbaren Hohlraum (40) eingeführt werden, dass dann der Ballon (12) über seinen Ballonanschluss (16) unter Überdruck gesetzt wird, so dass er sich an den Hohlraum (40) anlegt, und dass dann ein Scan über einen vorgegebenen Scanbereich (42) hinweg gestartet und durchgeführt wird, wobei aus der Verformung des Referenzmusters (26) die Form des Scanbereichs (42) des Hohlraums (40) berechnet wird.

16. Verfahren nach Anspruch 15, **dadurch gekennzeichnet, dass** ein erster Scan bei einem ersten Überdruck, bei welchem die Ballonfolie (14) an dem Hohlraum (40) anliegt, vorgenommen wird und ein zweiter Scan bei einem zweiten Überdruck, bei welchem die Ballonfolie (14) den Hohlraum (40) verformt, vorgenommen wird.

17. Verfahren nach Anspruch 15 oder 16, **dadurch gekennzeichnet, dass** eine Steuervorrichtung (32) der Scanvorrichtung (10) aus der Verformung des Referenzmusters (26) entsprechend der Verformung der Oberfläche des Hohlraums (40) die Nachgiebigkeit der Oberfläche und/oder die Veränderung der Oberfläche ermittelt.

18. Verfahren zum Betrieb einer Scanvorrichtung nach Anspruch 15, wobei die Scanvorrichtung einen Scankopf und eine Scankopfführung, die durch eine Öffnung in einem Hohlraum geführt wird, aufweist, **dadurch gekennzeichnet, dass** der Hohlraum (40) mit einem elastisch dehnbaren, sich über die Fläche betrachtet zwel-dimensional erstreckenden oder folienartigen Material abgedeckt ist, das an den Hohlraum (40) angedrückt wird, und wobei der Hohlraum (40) vor dem Scannen in seinem Volumen verkleinert wird.

19. Verfahren nach Anspruch 18, **dadurch gekennzeichnet, dass** der Hohlraum (40) kontrolliert hinsichtlich seines Volumens verkleinert wird und die Form des Referenzmusters (26) des folienartigen Materials erfasst und/oder gemessen wird.

20. Verfahren nach einem der Ansprüche 15 bis 19, **dadurch gekennzeichnet, dass** die Scanvorrichtung die Volumenvergrößerung des Ballons und/oder die Dehnung der Ballonfolie (14) beim Aufblasen erfasst und hierzu die Steuervorrichtung (32) die Vergrößerung des Referenzmusters (26) beim Aufblasen, ermittelt, wobei die Erfassung bevor die Ballonfolie (14) durch Anlage an den Scanbereich (42) verformt wird, und wobei nachdem die Ballonfolie durch Anlage an den Scanbereich verformt wird, durchgeführt wird, wobei hieraus die Reduktion der Stärke der Ballonfolie und/oder des Referenzmusters (26) errechnet wird.
